# EUROPEAN PATENT SPECIFICATION

(11) **EP 3 214 173 B1**
(45) Date of publication and mention of the grant of the patent: **02.10.2019**
(21) Application number: 16158090.7
(22) Date of filing: 01.03.2016
(51) Int. Cl.: C12N 9/42, C12P 19/02, C12P 19/14, C12P 7/10

(54) **USE OF A SUGAR TOLERANT BETA-GLUCOSIDASE**
VERWENDUNG VON ZUCKERTOLERANTER BETA-GLUCOSIDASE
UTILISATION D'UN BETA-GLUCOSIDASE TOLÉRANT LE SUCRE

(43) Date of publication of application: 06.09.2017
(73) Proprietor: Université de Liège, 4000 Liège (BE)
(72) Inventor: JOURDAN, Samuel, 4000 Liège (BE); RIGALI, Sébastien, 4000 Liège (BE)
(74) Representative: Bosch, Henry

(56) References cited:
- WO-A1-2016/044527
- US-A- 5 747 320
- DATABASE UniProt [Online] 24 November 2009 (2009-11-24), "RecName: Full=Beta-glucosidase {ECO:0000256|RuleBase:RU361175}; EC=3.2.1.21 {ECO:0000256|RuleBase:RU361175};", XP002760443, retrieved from EBI accession no. UNIPROT:C9Z448 Database accession no. C9Z448
- BIGNELL DAWN R D ET AL: "Streptomyces scabies 87-22 Contains a Coronafacic Acid-Like Biosynthetic Cluster That Contributes to Plant-Microbe Interactions", MOLECULAR PLANT-MICROBE INTERACTIONS, AMERICAN PHYTOPATHOLOGICAL SOCIETY, US, vol. 23, no. 2, 1 February 2010 (2010-02-01), pages 161-175, XP002674193, ISSN: 0894-0282, DOI: DOI:10.1094/MPMI-23-2-0161
- HELE TEUGJAS ET AL: "Selecting [beta]-glucosidases to support cellulases in cellulose saccharifica", BIOTECHNOLOGY FOR BIOFUELS, BIOMED CENTRAL LTD, GB, vol. 6, no. 1, 24 July 2013 (2013-07-24), page 105, XP021157200, ISSN: 1754-6834, DOI: 10.1186/1754-6834-6-105
- SOUZA FLAVIO HENRIQUE MOREIRA ET AL: "Gene cloning, expression and biochemical characterization of a glucose- and xylose-stimulated [beta]-glucosidase fromHumicola insolens", JOURNAL OF MOLECULAR CATALYSIS. B, ENZYMATIC, vol. 106, 21 April 2014 (2014-04-21), pages 1-10, XP028862043, ISSN: 1381-1177, DOI: 10.1016/J.MOLCATB.2014.04.007

## Description

### FIELD OF THE INVENTION

The present disclosure is generally directed to enzymes and in particular to the use of a polypeptide having sugar-tolerant beta-glucosidase activity for hydrolysis of a lignocellulosic substrate to yield glucose and/or other sugars.

### BACKGROUND OF THE INVENTION

Currently, the utilization of lignocellulosic biomass to produce monomer sugars that can be further fermented in ethanol or transformed in other high added-value molecules presents significant technical and economic challenges, and its success depends largely on the development of highly efficient and cost-effective biocatalysts (e.g. enzymes) for hydrolysis of pretreated lignocellulosic biomass. □

Lignocellulosic biomass pretreatment is a prerequisite for its efficient biological degradation. Indeed, cellulose fibrils are embedded in an amorphous matrix of lignin and hemicellulose that must be degraded to increase the accessibility of cellulose to enzymes involved in its hydrolysis. Classical pre-treatment methods include acid, base or organic solvents pretreatment, steam-, ammonia fiber- or CO₂ explosion, as well as wet-oxidation (Alvira et al, Bioresour. Technol. 2010, 101, 4851; Sorensen et al, Biomolecules 2013, 3, 612).

After pre-treatment, hydrolysis of lignocellulosic biomass classically involves sequential and synergistic actions of three main categories of enzymes, namely endoglucanases (EC 3.2.1.4), cellobiohydrolases (EC 3.2.1.91) and beta-glucosidases (EC 3.2.1.21). Endoglucanases rapidly decrease the degree of polymerization of lignocellulosic biomass substrate by randomly hydrolyzing the internal 1,4-beta-linkages of cellulose. Cellobiohydrolases further hydrolyze the cellulose polymer from the free ends, thereby releasing cellobiose that is finally degraded into glucose by beta-glucosidases (Wang et al, J. Chem. Technol. Biotechnol. 2013, 88, 491; Sorensen et al, Biomolecules 2013, 3, 612).

The hydrolysis reaction mechanism of beta-glucosidases comprises two catalytic steps, the second of which involves a base-catalyzed H₂O attack, resulting in the regeneration of the enzyme, and the release of two glucose residues.

It has been shown that cellobiohydrolases and endoglucanases are often inhibited by cellobiose. By reducing cellobiose accumulation, beta-glucosidases thus play a key role for an efficient hydrolysis of lignocellulosic biomass (Sorensen et al, Biomolecules 2013, 3, 612). However, beta-glucosidases are often themselves inhibited by their product glucose, making beta-glucosidase the rate-limiting enzyme in biomass hydrolysis (Andric et al, Biotechnol. Adv. 2010, 28, 308; Sorensen et al, Biomolecules 2013, 3, 612).

Another frequently reported unwanted event in lignocellulosic biomass hydrolysis is due to the transglycosylation activity of beta-glucosidases (Bohlin et al, Appl. Microbiol. Biotechnol. 2013, 97, 159). Indeed, whereas the hydrolysis of cellobiose by beta-glucosidase results in the formation of two molecules of glucose, transglycosylation results in the formation of one molecule of glucose and one trisaccharide (Teugjas et al, Biotechnology for Biofuels 2013, 6, 105), thereby decreasing the overall rate of hydrolysis. Hence oligosaccharides can be further resynthesized in presence of a second saccharide to the reaction mixture, which competes with the H₂O molecule, and reacts in its place with the first saccharide in a transglycosylation reaction. In the simplest transglycosylation reactions, the second saccharide is the substrate or the product itself (e.g. cellobiose or glucose).

Hence, the use of beta-glucosidases highly tolerant toward sugar, such as glucose, inhibition and having reduced transglycosylation activity is of great importance for an efficient conversion of pretreated lignocellulosic biomass to fermentable sugars.

Some glucose tolerant beta-glucosidases have been disclosed in the art. US5747320 discloses a glucose and cellobiose tolerant beta-glucosidase from *Candida peltata* NRRL Y-21603. This beta-glucosidase may be used in conjunction with cellulolytic enzymes for the treatment of lignocellulosic materials to convert cellulose to glucose. The enzyme possesses an inhibition constant, Ki (the concentration required to produce half maximum inhibition), with respect to glucose of about 1.4M, and crude preparations exhibit substantially no inhibition of activity at glucose concentrations less than or equal to about 12% (w/v). Moreover the enzyme does not have any transglycosylation activity. On the other hand, DE10219052 discloses a glucose-tolerant beta-glucosidase from *Microbispora bispora* NRRL 15568. The enzyme catalyzes conversion of cellobiose to glucose even in the presence of high concentrations of substrate and product.

During lignocellulosic biomass hydrolysis, other inhibiting compounds that can decrease the overall rate of hydrolysis also accumulate. Beside glucose, other sugars emanating from hemicellulose hydrolysis, such as mannose, xylose and galactose, have been shown to be strong inhibitors of lignocellulosic biomass hydrolysis by enzymes (Qing et al, Bioresource Technology 101, 2010, 9624).

Lignocellulosic biomass hydrolysis in sugars and their further fermentation in ethanol can be conducted in different vessels, or simultaneously in a process called simultaneous saccharification and fermentation (SSF). SSF is usually preferred due to its lower cost, the reduced contamination risk, and lower sugar inhibitory effects. However, there are still several drawbacks in SSF processes, such as cellulolytic enzyme inhibition by ethanol (Jorgensen et al, Biotechnology and Bioengineering, 2007, 96, 862).

Beta-glucosidases can catalyze the hydrolysis of a number of different substrates; hence the use of this enzyme in other applications than ethanol production is also possible. For instance, beta-glucosidases can be used to liberate aroma in plant-derived products through the hydrolysis of the glucoside precursors, especially monoterpenyl β-d-giucosides, thereby improving aroma of plant-derived beverages or food, such as wine or fruit juice (Baffi et al, Applied Biochemistry and Biotechnology, 2013, 169, 493).

During lignocellulosic biomass hydrolysis, the amount of the end-product glucose is increasing in the course of the reaction, and is generally not removed. Other sugars emanating from hemicellulose degradation also accumulate. In SSF processes, enzymes must also function under high alcohol (e.g. ethanol) concentrations. The same is true in the field of aroma improvement of plant-derived products, where biocatalysts must function in e.g. fruit juices or wine containing high glucose and/or ethanol concentrations.

There is therefore still an unmet need for beta-glucosidases that are highly tolerant to sugar and alcohol, and also exhibiting reduced transglycosylation activity.

### SUMMARY OF THE INVENTION

The genome of *Streptomyces scabies* (or *Streptomyces scabiei*) 87-22, a streptomycete bacterium species found in soils around the world, has been annotated in by Bignell et al (Molecular plant-microbe interactions, 2010, 23;2;161-75). The sequence of SEQ ID NO:2 described herein was published by National Center for Biotechnology Information (NCBI) with the Accession No. CBG72797 [synonyms: SCAB_RS27575, WP_013003368.1, or C9Z448 (C9Z448_STRSW)], and designated to be a putative beta-glucosidase. The enzyme has not been previously made in a recombinant form. The inventors have cloned and purified the enzyme and have confirmed that the enzyme has beta-glucosidase activity, i.e. is able to hydrolyze cellobiose into glucose. The enzyme has a mean Km of 0.77 mM, a mean Kcat of 400 min⁻¹, a Vₘₐₓ of 7.3 µmol.min⁻¹.mg⁻¹, an optimum temperature of between 30°C and 37°C and a pH optimum of about 7.5. The inventors have further demonstrated that the enzyme is capable of hydrolyzing other cello-oligosaccharides than cellobiose, namely cellotriose, cellotetraose, cellopentaose and cellohexaose. Having conducted extensive experiments and tests, the inventors have also surprisingly found that the beta-glucosidase from *Streptomyces scabies* 87-22 is not only tolerant to sugar inhibition but is also activated by all the tested sugars. It is activated by glucose or xylose concentrations up to 1.6M, and is further activated by fructose, galactose, mannose and sorbitol. This is in contrast with the beta-glucosidase disclosed in US5747320 which is tolerant to, but not activated by, glucose. Moreover, the inventors have shown that increasing concentrations of ethanol (up to about 20%) also enhance the activity of the enzyme. At ethanol concentrations greater than 20%, the enzyme is still active. Hence the enzyme is also ethanol tolerant. The inventors also surprisingly found that the enzyme does not have any transglycosylation activity under high glucose concentrations.

Hence, the beta-glucosidase of *Streptomyces scabies* 87-22 can advantageously be used for highly effective hydrolysis of lignocellulosic substrates into glucose that can be further or simultaneously fermented in ethanol or transformed in other high added-value molecules. The enzyme can also advantageously be used to liberate aroma in plant-derived products. The present invention hence offers a more effective and promising alternative to the enzymes or processes already described in the art.

### BRIEF DESCRIPTION OF THE FIGURES

The present invention is illustrated by the following figures which are to be considered for illustrative purposes only and in no way limit the invention to the embodiments disclosed therein:
**Figure 1**: represents the level of purity of the recombinant His-tagged beta glucosidase of *Streptomyces scabies* 87-22 (named BgIC).
**Figure 2****:** represents thin layer chromatography analysis of cellobiose and cello-oligosaccharides hydrolysis by purified BglC. 6.25 mM of cello-oligosaccharides were incubated with pure BglC (0.4 µM) at 30°C for 0, 15, 30 and 60 min. M: standard cello-oligossacharides (cellopentaose and cellohexaose cannot migrate under these conditions and therefore are stacked at the initial spot).
**Figure 3****:** represents the effect of temperature and pH on BglC activity.
**Figure 4****:** represents the effect of D-glucose and D-xylose on BglC activity. The activity measured without any additive was fixed to 100% (Control).
**Figure 5****:** represents time course of cellobiose hydrolysis by BglC. Glucose accumulation is also shown.
**Figure 6****:** represents the effect of ethanol on BglC activity.
**Figure 7**: represents the effect of NaCl on BglC activity. 100% was fixed as the activity measured without any additive (control).

### DETAILLED DESCRIPTION OF THE INVENTION

As used herein, the singular forms "a", "an", and "the" include both singular and plural referents unless the context clearly dictates otherwise. By way of example, "a kit-of-parts" refers to one or more than one kit-of-parts.

The terms "comprising", "comprises" and "comprised of" as used herein are synonymous with "including", "includes" or "containing", "contains", and are inclusive or open-ended and do not exclude additional, non-recited members, elements or method steps. Said terms also encompass the specific embodiments "consisting essentially of" and "consisting of".

The recitation of numerical ranges by endpoints includes all numbers and fractions subsumed within the respective ranges, as well as the recited endpoints.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

According to a first aspect, provided herein is a use of a polypeptide having sugar-tolerant beta-glucosidase activity for hydrolysis of a lignocellulosic substrate to yield glucose and/or other sugars,
characterized in that said polypeptide is selected from the group consisting of:
a) a polypeptide comprising or consisting of an amino acid sequence at least 50%, preferably at least 60%, more preferably at least 70%, even more preferably at least 80%, still even more preferably at least 90%, most preferably at least 95%, such as 96%, 97%, 98% or 99%, even most preferably 100% identical to the amino acid sequence of SEQ ID NO: 2,
b) a polypeptide which is encoded by a polynucleotide at least 50%, preferably at least 60%, more preferably at least 70%, even more preferably at least 80%, still even more preferably at least 90%, most preferably at least 95%, such as 96%, 97%, 98% or 99%, even most preferably 100% identical to SEQ ID NO: 1,
c) a polypeptide which is encoded by a polynucleotide which hybridizes under at least medium stringency conditions, preferably under at least high stringency conditions, with the polynucleotide of SEQ ID NO: 1, or a complementary strand thereof, and
d) a fragment of a), b), or c) having beta-glucosidase activity;
and said polypeptide or fragment thereof is obtained from a *Streptomyces* bacterium.

The term "polypeptide" refers to a molecule comprising amino acid residues linked by peptide bonds and containing more than five amino acid residues. The term "protein" as used herein is synonymous with the term "polypeptide" and may also refer to two or more polypeptides. Thus, the terms "protein" and "polypeptide" can be used interchangeably. Polypeptides may optionally be modified (e.g., glycosylated, phosphorylated, acylated, farnesylated, prenylated, sulfonated, and the like) to add functionality. Polypeptides exhibiting activity in the presence of a specific substrate under certain conditions may be referred to as enzymes.

A "polynucleotide" is defined herein as a nucleotide polymer comprising at least 5 nucleotide units. A nucleotide refers to RNA or DNA. The terms "nucleic acid" and "polynucleotide" are used interchangeably herein.

The term "complementary strand" can be used interchangeably with the term "complement". The complementary strand of a polynucleotide can be the complement of a coding strand or the complement of a non-coding strand. When referring to double-stranded nucleic acids, the complement of a polynucleotide encoding a polypeptide refers to the complementary strand of the strand encoding the amino acid sequence or to any nucleic acid molecule containing the same.

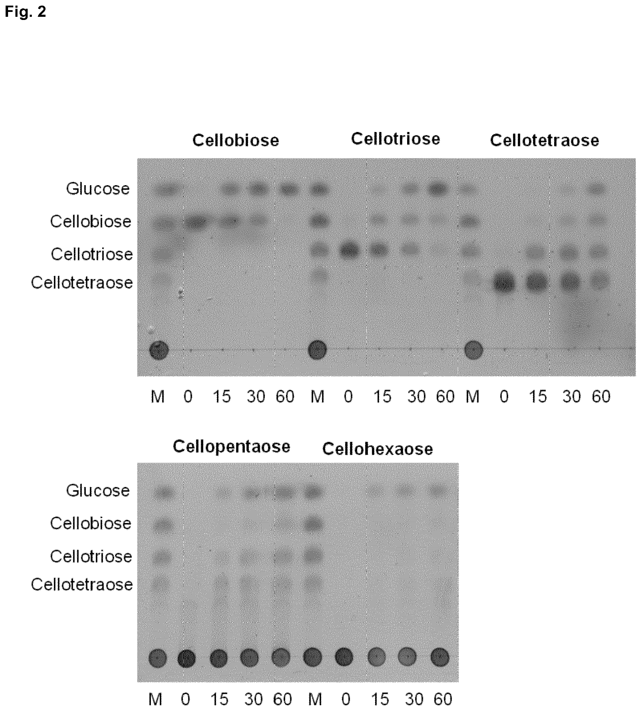

The term "beta-glucosidase" refers to a beta-D-glucoside glucohydrolase of E.C. 3.2.1.21. The term "beta-glucosidase activity" therefore refers the capacity of catalyzing the hydrolysis of beta-D-glucose or cellobiose to release D-glucose. Beta-glucosidase activity may be determined using a cellobiase assay, for example, which measures the capacity of the enzyme to catalyze the hydrolysis of a cellobiose substrate to yield D-glucose, as described in Figure 2 of the present disclosure. As used herein, the term "beta-glucosidase activity" also encompasses capacity of catalyzing the hydrolysis of other cello-oligosaccharides than cellobiose, for example, but not limited to, one or more of cellotriose, cellotetraose, cellopentaose or cellohexaose, as described in figure 2 of the present disclosure. The skilled person is well aware that beta-glucosidases may be referred to by different names, or synonyms.

The terms "tolerant" or "tolerant manner", when referring to a beta-glucosidase activity in the presence of a compound such as sugar, DTT, EDTA, urea, NaCl or ethanol, refers to the capacity of said beta-glucosidase of catalyzing the hydrolysis of a lignocellulosic substrate into glucose in the presence of said compound, or when the concentration of said compound increases during hydrolysis. Alternatively, this term also refers to an increased/enhanced capacity of catalyzing the hydrolysis of a lignocellulosic substrate when concentration of said compound increases during hydrolysis. By "increased/enhanced capacity", we mean a higher activity upon compound accumulation in the reaction medium compared to the initial compound concentration present at the beginning of the hydrolysis. Tests for measuring tolerance of a beta-glucosidase toward a compound such as sugar, DTT, EDTA, urea, NaCl or ethanol are well known to those skilled in the art (see for instance the example section of this application).

The term "sugar" refers to short chain soluble carbohydrates, which may be divided in different subgroups, such as monosaccharides (such as glucose, galactose, fructose, mannose, and xylose), disaccharides (such as sucrose, lactose, maltose and trehalose) and polyols (such as sorbitol and mannitol). Preferred sugars are selected from the group comprising: glucose, xylose, fructose, galactose, mannose, and sorbitol. Preferred sugars are glucose and/or xylose.

The term "lignocellulosic substrate" refers to any substrate comprising cellulose and/or hemicellulose (optionally also lignin) and/or degradation products thereof. Non-limiting examples of cellulose degradation products are cellodextrins and/or lower molecular weight cellulose byproducts such as, but non-limited to, cellobiose, cellotriose, cellotetraose, cellopentaose or cellohexaose. Preferably the lignocellulosic substrate comprises one or more of cellobiose, cellotriose, cellotetraose, cellopentaose or cellohexaose. More preferably the lignocellulosic substrate comprises cellobiose.

Lignocellulosic substrate may include, but is not limited to, leaves and stalks of both woody and non-woody plants. The term "woody" is used herein both in the botanical sense to mean "comprising wood" that is, composed of extensive xylem tissue as found in trees and shrubs. Accordingly, "non-woody" refers to materials lacking these characteristics. Non-limiting examples of lignocellulosic substrate are crops such as starch crops (e.g., corn, wheat, rice or barley), sugar crops (e.g., sugarcane, energy cane or sugar beet), forage crops (e.g., grasses, alfalfa, or clover), and oilseed crops (e.g., soybean, sunflower, or safflower); wood products such as trees, shrubs, and wood residues (e.g., sawdust, bark or the like from forest clearings and mills); waste products such as municipal solid waste (e.g., paper, food and yard wastes or wood), and process waste; and aquatic plants such as algae, water weed, water hyacinth, or reed and rushes. Lignocellulosic substrate from non-woody plants in agriculture may be derived from monocotyledonous plants, and especially grassy species belonging to the family Gramineae. Of primary interest are gramineous agricultural residues; that is, the portion of grain-bearing plants that remain after harvesting the seed. Illustrative of such residues, without limitation thereto, are wheat straw, oat straw, rice straw, barley straw, rye straw, flax straw, sugar cane, corn stover, corn stalks, corn cobs, corn husks, and the like. Also included within this definition are grasses not conventionally cultivated for agricultural purposes, such as prairie grasses (e.g. big bluestem, little bluestem, Indian grass), switchgrass, gamagrass, and foxtail. In some embodiments, the lignocellulosic substrate comprises corn kernel, barley kernel, milo kernel, wheat kernel or rice kernel.

"Percent (%) identical to" or "percent (%) sequence identity" with respect to the amino acid or polynucleotide sequences identified herein is defined as the percentage of amino acid residues or nucleotides in a candidate sequence that are identical with the amino acid residues of SEQ ID NO:2 or with nucleotides of sequence NO:1, after aligning the sequences and introducing gaps, if necessary, to achieve the maximum percent sequence identity, and not considering any conservative amino acids or nucleotide substitutions as part of the sequence identity. Preferably said alignment is done over the whole length of the reference sequence. Conservative amino acid substitutions refer to those that preserve the general charge, hydrophobicity/hydrophilicity, and/or steric bulk of the amino acid being substituted. Non-limiting examples of conservative amino acid substitutions are those between the following groups: Gly/Ala, Val/Ile/Leu, Lys/Arg, Asn/Gln, Glu/Asp, Ser/Cys/Thr, and Phe/Trp/Tyr. Conservative nucleotide substitutions refer to those that preserve the general charge, hydrophobicity/hydrophilicity, and/or steric bulk of the amino acid being encoded by the substituted nucleotide. Methods for aligning sequences and determining sequence identity are known to the skilled person and may be performed without undue experimentation. See, for example, Ausubel et al., eds. (1995) Current Protocols in Molecular Biology, Chapter 19 (Greene Publishing and Wiley-Interscience, New York). A number of algorithms are available for aligning sequences and determining sequence identity and include, for example, the BLASTP, BLASTN, and BLASTX algorithms (see Altschul et al. (1990) J. Mol. Biol. 275:403-410). Those skilled in the art can determine appropriate parameters for measuring alignment, including algorithms needed to achieve maximal alignment over the length of the sequences being compared. Preferably, the sequence identity is determined using the default parameters determined by the program. Specifically, sequence identity can be determined by using the BlastP or BlastN programs of the NCBI.

The term "hybridization" or "hybridizes" means the pairing of substantially complementary strands of oligomeric compounds, such as polynucleotides. Hybridization may be performed under medium or high stringency conditions. High stringency condition refers to high hybridization temperature and low salt in hybridization buffers, whereas medium stringency refers to lower temperature and higher salt in hybridization buffers. Preferably medium stringency hybridization conditions comprise hybridizing in 6X sodium chloride/sodium citrate (SSC) at about 45°C, followed by one or more washes in 0.2X SSC, 0.1 % SDS at 60°C, and high stringency hybridization conditions comprise hybridizing in 6X SSC at about 45°C, followed by one or more washes in 0.2X SSC, 0.1 % SDS at 65°C.

To detect hybridization of a polynucleotide to its target polynucleotide sequence (e.g. the polynucleotide of SEQ ID NO: 1, or a complementary strand thereof), the polynucleotide may be labeled with a molecular marker, such as, but not limited to, a radioactive or a fluorescent molecule. Commonly used markers are ³²P (a radioactive isotope of phosphorus incorporated into the phosphodiester bond in the polynucleotide) or digoxigenin, which is a non-radioactive, antibody-based marker. Hybridization of a polynucleotide to its target may be then detected by visualizing the hybridized polynucleotide via autoradiography or other imaging techniques.

A "fragment having beta-glucosidase activity" may be derived from a parent polypeptide, which may be any one or more of:
a) a polypeptide comprising or consisting of an amino acid sequence at least 50%, preferably at least 60%, more preferably at least 70%, even more preferably at least 80%, still even more preferably at least 90%, most preferably at least 95%, such as 96%, 97%, 98% or 99%, even most preferably 100% identical to the amino acid sequence of SEQ ID NO: 2,
b) a polypeptide which is encoded by a polynucleotide at least 50%, preferably at least 60%, more preferably at least 70%, even more preferably at least 80%, still even more preferably at least 90%, most preferably at least 95%, such as 96%, 97%, 98% or 99%, even most preferably 100% identical to SEQ ID NO: 1,
c) a polypeptide which is encoded by a polynucleotide which hybridizes under at least medium stringency conditions, preferably under at least high stringency conditions, with the polynucleotide of SEQ ID NO: 1, or a complementary strand thereof.

The parent polypeptide may have been truncated either in the N-terminal region, or the C-terminal region, or in both regions to generate a fragment of the parent polypeptide. For the purpose of the present disclosure, a fragment having beta-glucosidase activity must have at least 20%, preferably at least 30%, 40%, 50%, or more preferably, at least 60%, 70%, 80%, or even more preferably at least 90% of the beta-glucosidase activity of that of the parent polypeptide.

Preferably fragments having beta-glucosidase activity are at least 20 amino acid residues in length (e.g., at least 20 amino acid residues, at least 40 amino acid residues, at least 60 amino acid residues, at least 80 amino acid residues, at least 100 amino acid residues, at least 120 amino acid residues, at least 140 amino acid residues, at least 160 amino acid residues, at least 180 amino acid residues, at least 200 amino acid residues, at least 220 amino acid residues, at least 240 amino acid residues, at least 260 amino acid residues, at least 280 amino acid residues, at least 300 amino acid residues, at least 320 amino acid residues, at least 340 amino acid residues, or at least 360 amino acid residues in length or longer). Such fragments suitably retain the active site of the parent polypeptides but may have deletions of non-critical amino acid residues or conservative amino acid substitutions. The activity of fragments can be readily determined using the assays described herein, for example those described in figure 2, or by other assays known in the art.

The term *"Streptomyces* bacterium" refers to bacterium of the Streptomycetaceae family found in soils and other sediments around the world. Examples of *Streptomyces* bacterium includes, with no limitation, *Streptomyces scabies or scabiei, S. acidiscabies, S. europaeiscabiei, S. luridiscabiei, S. niveiscabiei, S. puniciscabiei, S. reticuliscabiei, S. stelliscabiei, S. turgidiscabies* or *S*. *ipomoeae.* In a preferred embodiment, the Streptomyces bacterium is *Streptomyces scabies or scabiei,* more preferably *Streptomyces scabies* 87-22. *Streptomyces scabies* refers to bacterial strains, which have the identifying characteristics of culture deposit of any one or more of ATCC numbers 49173, 33282, 10246, 700528, or 33281. *Streptomyces scabies* 87-22 refers to a bacterial strain described in Loria et al, Biochem. Cell. Biol. 1995, 85 (5): 537-541.

The terms "kcat" and "Km" and Vₘₐₓ" are known to those skilled in the art and are described in Enzyme Structure and Mechanism, 2nd ed. (Ferst, W.H. Freeman: NY, 1985; pp 98-120).

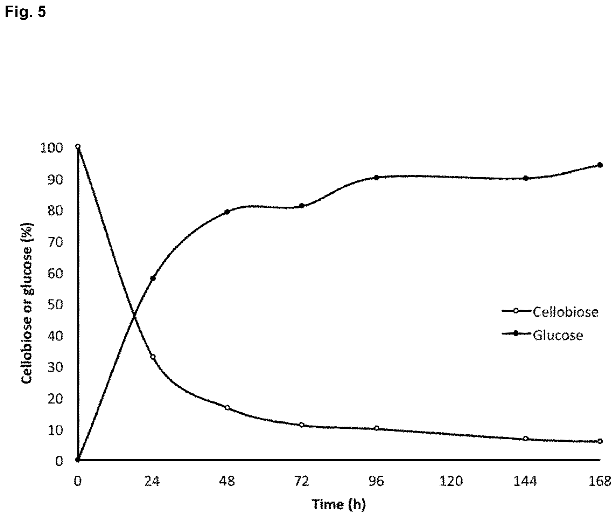

The term "transglycosylation activity" of a beta-glucosidase refers to its capacity to form one glucose molecule (instead of two) and one oligosaccharide from its substrate (e.g. cellobiose). Transglycosylation activity is often increased under high substrate and/or product concentration. Tests for measuring transglycosylation activity of a beta-glucosidase are well known to those skilled in the art (see for example in US5716812, incorporated by reference herein, or in figure 5 of this application). In a preferred embodiment, transglycosylation occurs between glucose and cellobiose.

Fermentation of glucose and/or other sugars in alcohol, such as ethanol, may be done using conventional techniques. Many techniques are well known to the skilled person, and are suitable for use herein. As an example only, the hydrolyzate containing the glucose and/or other sugars may be contacted with an appropriate microorganism under conditions effective for the fermentation of the glucose to alcohol, such as ethanol. Preferably the microorganism is an ethanologen microorganism, i.e. a microorganism with the ability to convert glucose and/or other sugars to ethanol. This fermentation may be separate from and follow the enzymatic hydrolysis of the lignocellulosic substrate, or the hydrolysis and fermentation may be concurrent and conducted in the same vessel in a process called simultaneous saccharification and fermentation (SSF). Details of the various fermentation techniques, conditions, and suitable microorganisms have been described in the art.

After completion of the fermentation, the ethanol may be recovered and optionally purified or distilled and used for example as drinking alcohol or fuel (e.g., a biofuel such as a bioethanol, biobutanol, biomethanol, biopropanol, biodiesel, jet fuel, or the like).

In other embodiments, lignocellulosic substrate hydrolyzed by the beta-glucosidase of the disclosure can also be made into a commodity chemical (e.g., ascorbic acid, isoprene, 1,3-propanediol), lipids, amino acids, polypeptides, and enzymes, via fermentation and/or chemical synthesis.

According to a second aspect, provided herein is a use of a host cell comprising and/or secreting a polypeptide having sugar-tolerant beta-glucosidase activity for hydrolyzing a lignocellulosic substrate in a sugar-tolerant manner, yielding glucose and/or other sugars,
characterized in that said polypeptide is selected from the group consisting of:
a) a polypeptide comprising or consisting of an amino acid sequence at least 50%, preferably at least 60%, more preferably at least 70%, even more preferably at least 80%, still even more preferably at least 90%, most preferably at least 95%, such as 96%, 97%, 98% or 99%, even most preferably 100% identical to the amino acid sequence of SEQ ID NO: 2,
b) a polypeptide which is encoded by a polynucleotide at least 50%, preferably at least 60%, more preferably at least 70%, even more preferably at least 80%, still even more preferably at least 90%, most preferably at least 95%, such as 96%, 97%, 98% or 99%, even most preferably 100% identical to SEQ ID NO: 1,
c) a polypeptide which is encoded by a polynucleotide which hybridizes under at least medium stringency conditions, preferably under at least high stringency conditions, with the polynucleotide of SEQ ID NO: 1, or a complementary strand thereof, and
d) a fragment of a), b), or c) having beta-glucosidase activity;
and said polypeptide or fragment thereof is obtained from a *Streptomyces* bacterium.

As used herein, a "host cell comprising and/or secreting a polypeptide" is an organism into which an expression vector, phage, virus, or other DNA construct comprising a polynucleotide encoding said polypeptide has been introduced. Exemplary host strains are microbial cells (e.g., bacteria, filamentous fungi, and yeast) capable of expressing and/or secreting the polypeptide of interest. The term "host cell" also includes protoplasts created from cells.

Host cells useful in the present invention are generally prokaryotic or eukaryotic hosts, including any transformable organism in which expression and/or secretion can be achieved. Specifically, host strains may be *Bacillus subtilis, Streptomyces lividans, Streptomyces scabies, Escherichia coli, Trichoderma reesei, Saccharomyces cerevisiae,* or *Aspergillus niger.* In certain embodiments, the host cell may be an ethanologen microorganism, which may be, for example, a yeast such as *Saccharomyces cerevisiae* or an ethanologen bacterium such as *Zymomonas mobilis.* When *Saccharomyces cerevisiae* or *Zymomonas mobilis* is used as the host cell, and if the beta-glucosidase gene is not made to secret from host cell but is expressed intracellularly, a cellobiose transporter gene can be introduced into the host cell in order to allow the intracellularly expressed beta-glucosidase to act upon the cellobiose substrate and liberate glucose, which will then be metabolized subsequently or immediately by the microorganisms and converted into ethanol.

In a preferred embodiment, the invention relates to the use of a host cell comprising and/or secreting the polypeptide of the invention, wherein said polypeptide further comprises a signal peptide sequence.

A "signal peptide sequence" refers to a sequence of amino acids bound to the N-terminal portion of a polypeptide, and which facilitates the secretion of the mature form of the polypeptide from the cell. The mature form of the extracellular polypeptide lacks the signal sequence which is cleaved off during the secretion process.

According to a third aspect, the invention provides a kit-of-parts for hydrolyzing a lignocellulosic substrate in a sugar-tolerant manner, comprising: a polypeptide having sugar-tolerant beta-glucosidase activity, and one or more other cellulases, characterized in that said polypeptide is selected from the group consisting of:
a) a polypeptide comprising or consisting of an amino acid sequence at least 50%, preferably at least 60%, more preferably at least 70%, even more preferably at least 80%, still even more preferably at least 90%, most preferably at least 95%, such as 96%, 97%, 98% or 99%, even most preferably 100% identical to the amino acid sequence of SEQ ID NO: 2,
b) a polypeptide which is encoded by a polynucleotide at least 50%, preferably at least 60%, more preferably at least 70%, even more preferably at least 80%, still even more preferably at least 90%, most preferably at least 95%, such as 96%, 97%, 98% or 99%, even most preferably 100% identical to SEQ ID NO: 1,
c) a polypeptide which is encoded by a polynucleotide which hybridizes under at least medium stringency conditions, preferably under at least high stringency conditions, with the polynucleotide of SEQ ID NO: 1, or a complementary strand thereof, and
d) a fragment of a), b), or c) having beta-glucosidase activity;
and said polypeptide or fragment thereof is obtained from a *Streptomyces* bacterium.

The term "kit-of-parts" (or kit) as used herein refers to any combination of reagents or apparatus than can be used, simultaneously, separately or sequentially, for hydrolyzing a lignocellulosic substrate in a sugar tolerant manner. Preferably, the kit of parts is provided in a format that is convenient for the end user. Suitable packaging and instructions may be provided. The instructions may be provided in printed form or in the form of an electronic medium such as a floppy disc, CD, or DVD, or in the form of a website address where such instructions may be obtained.

The term "cellulase" refers to any enzyme capable of catalyzing the decomposition of cellulose and of some related polysaccharides. Non-limiting examples of cellulases are endocellulases or endoglucanases, exocellulases or cellobiohydrolases, cellobiases or beta-glucosidases, oxidative cellulases and cellulose phosphorylases. As used herein, the term "cellulase" also encompasses hemicellulases, such as, but non-limited to, xylanases, beta-xylosidases or L-arabinofuranosidases. The skilled person is well aware that cellulases or hemicellulases may be referred to by different names, or synonyms.

According to a fourth aspect, the invention provides a method for hydrolyzing a lignocellulosic substrate in a sugar-tolerant manner, comprising: contacting the lignocellulosic substrate with an effective amount of a polypeptide having sugar-tolerant beta-glucosidase activity, with a host cell comprising and/or secreting a polypeptide having sugar-tolerant beta-glucosidase activity, or with the kit-of-parts of the invention, to yield glucose and/or other sugars,
characterized in that said polypeptide is selected from the group consisting of:
a) a polypeptide comprising or consisting of an amino acid sequence at least 50%, preferably at least 60%, more preferably at least 70%, even more preferably at least 80%, still even more preferably at least 90%, most preferably at least 95%, such as 96%, 97%, 98% or 99%, even most preferably 100% identical to the amino acid sequence of SEQ ID NO: 2,
b) a polypeptide which is encoded by a polynucleotide at least 50%, preferably at least 60%, more preferably at least 70%, even more preferably at least 80%, still even more preferably at least 90%, most preferably at least 95%, such as 96%, 97%, 98% or 99%, even most preferably 100% identical to SEQ ID NO: 1,
c) a polypeptide which is encoded by a polynucleotide which hybridizes under at least medium stringency conditions, preferably under at least high stringency conditions, with the polynucleotide of SEQ ID NO: 1, or a complementary strand thereof, and
d) a fragment of a), b), or c) having beta-glucosidase activity;
and said polypeptide or fragment thereof is obtained from a Streptomyces bacterium.

In a preferred embodiment, the method further comprises pretreating the lignocellulosic substrate with acid (e.g., HCl, H₂SO₄, or H₃PO₄) and/or base (e.g., NaOH, or NH₄ OH) and/or organic solvants (e.g., ethanol, methanol, ethylene glycol, butanol, phenol) and/or mechanical or other physical means. In some embodiments, the mechanical means may include, but is not limited to, pulling, pressing, crushing, grinding, and other means of physically breaking down the lignocellulosic substrate into smaller physical forms. Other physical means may also include, for example, using steam or other pressurized fume or vapor to "loosen" the lignocellulosic substrate in order to increase accessibility by the enzymes to the cellulose and/or hemicellulose. In certain embodiments, the method of pretreatment may also involve enzymes that are capable of breaking down the lignin of the lignocellulosic substrate.

Further, the disclosure provides a method for obtaining aroma in a plant-derived product in a sugar-tolerant manner, comprising: contacting the plant-derived product with an effective amount of a polypeptide having sugar-tolerant beta-glucosidase activity or with a host cell comprising and/or secreting a polypeptide having sugar-tolerant beta-glucosidase activity,
characterized in that said polypeptide is selected from the group consisting of:
a) a polypeptide comprising or consisting of an amino acid sequence at least 50%, preferably at least 60%, more preferably at least 70%, even more preferably at least 80%, still even more preferably at least 90%, most preferably at least 95%, such as 96%, 97%, 98% or 99%, even most preferably 100% identical to the amino acid sequence of SEQ ID NO: 2,
b) a polypeptide which is encoded by a polynucleotide at least 50%, preferably at least 60%, more preferably at least 70%, even more preferably at least 80%, still even more preferably at least 90%, most preferably at least 95%, such as 96%, 97%, 98% or 99%, even most preferably 100% identical to SEQ ID NO: 1,
c) a polypeptide which is encoded by a polynucleotide which hybridizes under at least medium stringency conditions, preferably under at least high stringency conditions, with the polynucleotide of SEQ ID NO: 1, or a complementary strand thereof, and
d) a fragment of a), b), or c) having beta-glucosidase activity.

Plant-derived products contain aroma components, and their inherent aroma properties can be released by degrading enzymes, e.g. turning a non-volatile aroma component into its volatile form. Beta-glucosidases can assist in the liberation of aroma components from plant-derived products such as fruit juices or wines. Moreover, in the case of wine, the liberation of aroma compounds provides wines with a more consistent flavor, thus reducing or eliminating the undesirable effect of "poor vintage years".

The term "aroma components", also referred to as odorant, aroma, fragrance, or flavor and the like, refers to a chemical compound that have a smell or odor. A chemical compound has a smell or odor when it is sufficiently volatile to be transported to the olfactory system.

As used herein, a "plant-derived product" refers to any material derived from plants, such as, but non-limited to, fruits and fruits juices such as grapes and grapes juice, alcoholic beverages such as wine, beer or their derivatives (for example all drinks containing wine or beer), or aromatic and/or flowering plants and their derivatives, such as tea or tobacco.

Methods for obtaining aroma components in a plant-derived product have been described in the art, for example in WO 89/08404, which is incorporated herein by reference. As an example only, beta-glucosidase can be used to cleave the terpene aglycone-carbohydrate link bond of terpene monoglucosides contained in the plant-derived product, thereby liberating terpenols, odoriferous volatile substances.

In a preferred embodiment, e.g. in case the plant-derived product contains mainly terpene diglycosides, the method can be performed in two-stage using an alpha-arabinosidase and/or an alpha-rhamnosidase for the first stage, and a beta-glucosidase for the second stage. During stage 1, actions of alpha-arabinosidase and/or alpha-rhamnosidase liberate the corresponding terpene monoglucosides by cleavage at the (1->6) glycoside bond. In the second stage beta-glucosidase provides for the liberation of the terpenols by cleavage of the terpene aglycone-carbohydrate link bond.

Non-limiting examples of aroma components obtained by the method of the invention are terpenols, such as geraniol, linalool, nerol and the like, and terpene polyols and alcohols such as phenyl ethyl and benzyl alcohol, or the like.

Methods to detect and quantify aroma components obtained by the method of the invention are well-known in the art and include, with no limitation, Gas-Chromatography (GC), Gas-Chromatography-lined Mass Spectrometry (GC/MS), Liquid Chromatography-tandem mass spectrometry (LC/MS), Ion Mobility Spectrometry/Mass Spectrometry (IMS/MS), Proton Transfer Reaction Mass-Spectrometry (PTR-MS), Electronic Nose device, quartz crystal microbalance or chemically sensitive sensors.

The present invention is further illustrated by the following examples, which do not limit the scope of the invention in any way.

### EXAMPLES

### Materials and methods

### Production and purification of histidine-tagged recombinant beta-glucosidase of Streptomyces scabies 87-22.

The open reading frame encoding SCAB57721 (hereafter named BgIC) was amplified by PCR using primers *scab_57721_*+3*_Ndel* (TTCATATGCCTGAACCCGTGAATCCGG) and *scab_57721_*+1458_*Hind*III (TT*AAGCTT*TGGTCCGTCGCTGCCCTACG). The corresponding PCR product was subsequently cloned into the pJET1.2/blunt cloning vector, yielding pSAJ021. After DNA sequencing to verify the correct amplification of *scab57721,* an *Nde*I*-Hind*III DNA fragment was excised from pSAJ021 and cloned into pET-28a digested with the same restriction enzymes leading to pSAJ022. All plasmids used and generated are listed in Table 1.

**Table 1. Bacterial strains and plasmids used in this study**

| **Plasmids and strains** | **Description ^{†}** | **Source or reference** |
|---|---|---|
| **Plasmids or cosmids** | | |
| pJET1.2/blunt | *E. coli* plasmid used for high-efficiency cloning of PCR products (Amp^{R}) | Thermo Scientific |
| pET28a | Expression vector used to produce N-terminal His-tagged protein in *E. coli* (Kan^{R}) | Novagen |
| pSAJ021 | pJET1.2 derivative containing the *scab57721* coding sequence (Amp^{R}) | This application |
| pSAJ022 | pET28a derivative containing the *scab57721* coding sequence inserted into *Nde*I and *Hind*III restriction sites (Amp^{R}) | This application |

| ***E. coli* strains** | | |
|---|---|---|
| DH5α | General cloning host | Gibco-BRL |
| Rosetta (DE3) | *E. coli* strain used to express a protein from pET-vectors (Cml^{R}) | Novagen |

| ***Streptomyces* strains** | | |
|---|---|---|
| 87-22 | *S. scabies* wild type strain | Loria et al 1995 |

| | | |
|---|---|---|
| † Cml^{R}, chloramphenicol resistance; Kan^{R}, kanamycin resistance; Amp^{R}, ampicillin resistance | | |

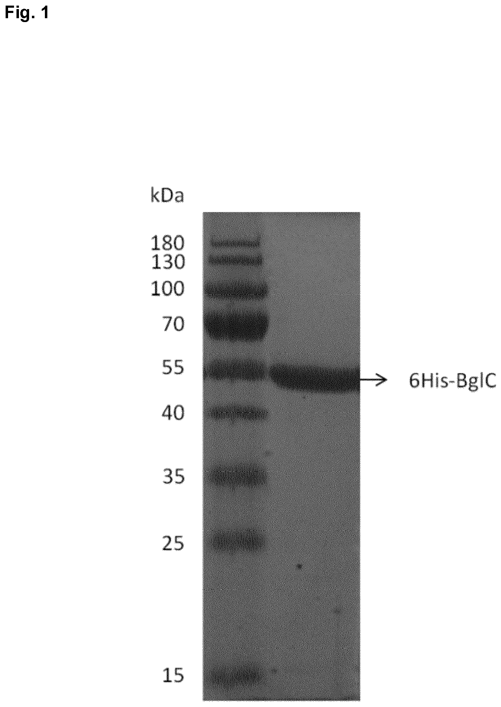

*E. coli* Rosetta (DE3) cells carrying pSAJ022 were grown at 37°C in 250 ml LB medium containing 100 µg/ml of kanamycin until the culture reached an optical density at 600 nm (OD₆₀₀) of 0.6. Production of 6His-tagged BglC (6His-BglC) was induced overnight (∼20 h) at 16°C by addition of 1 mM isopropyl-β-D-thiogalactopyranoside (IPTG). Cells were collected by centrifugation and ruptured by sonication in lysis buffer (100 mM Tris-HCI buffer; pH 7.5; NaCl 250 mM; Imidazol 20 mM supplemented with the EDTA-free complete protease inhibitor cocktail (Roche)). Soluble proteins were loaded onto a pre-equilibrated Ni²⁺-nitrilotriacetic acid (NTA)-agarose column (5-ml bed volume), and 6His-BglC eluted within the range of 100 to 150 mM imidazole. Fractions containing the pure protein were pooled (Figure 1) and dialyzed overnight in 50 mM HEPES; pH 7.5.

### Enzyme assays

Relative enzyme activity was determined using *p*-nitrophenyl-β-D-glucopyranoside (*p*-NPβG) as substrate. The reaction mixture (200 µL) containing 50 mM HEPES buffer (pH 7.5), 0.2 µM of purified BglC and the tested reagent was incubated 10 minutes at 25°C before addition of 1 mM *p*-NPβG. The reaction was carried out at 25°C for 2 minutes and stopped by addition of 100µL of 2 M Na₂CO₃. All assays were performed under these conditions, unless otherwise indicated. The release of p-nitrophenol (*p*-NP) was measured at 405 nm with a TECAN infinite ® 200 PRO. The activity assayed in absence of the tested reagent was recorded as 100%.

### Temperature and pH

The optimal temperature was determined by measuring the relative enzyme activity of BglC in HEPES 50 mM pH 7.5 at 20, 25, 30, 37 and 42 °C. To measure the effect of pH on activity of BgIC, relative activity was essayed in the range of pH 5.0 - 6.5 (50 mM MES buffer), pH 7.0 - 8.5 (50 mM HEPES buffer) and pH 9 - 10 (50 mM CHES buffer) at 25°C.

### Substrate specificity

The cleavage ability of BglC was tested against different cello-oligosacchardides (cellobiose, cellotriose, cellotetraose, cellopentaose and cellohexaose (Megazyme; Ireland)) or different disaccharides (lactose, saccharose, maltose, threalose and turanose). Reaction mixtures (100 µL) containing 50 mM HEPES buffer pH 7.5, 0.4 µM of purified BgIC, 6.25 mM of cello-oligosaccharides or 12.5 mM of disaccharides were incubated at 30 °C. 15 µL of each sample were collected at 0, 15, 30 and 60 min than heated at 98°C for 5 min to stop the reaction. Each sample was spotted onto aluminum-backed Silica gel plate (Sigma). The plates were developed with chloroform-methanol-acetic acid-water solvent (50:50:15:5, vol/vol), air dried, dipped in 5 % H₂SO₄ in ethanol and finally heated over a hot plate until visualization of carbohydrate spots.

### Kinetic analysis

Kinetic parameters of BglC (*Km* and *kcat*) were determined by measuring the glucose released at various cellobiose concentrations in 50 mM HEPES buffer pH 7.5 at 26°C. Reaction time of 7 min was chosen to ensure initial rates of hydrolysis. The glucose released was determined using the D-Glucose HK Assay Kit from Megazyme (Ireland). Data were fitted to the Henri-Michaelis-Menten equation using the GraphPad Prism 5 software.

### HPLC analysis

Glucose and cellobiose analysis was performed by HPLC on an Aminex HPX-87P Column (300 x 7.8 mm) heated to 80°C with H₂O as eluent (flow rate 0.6 mL/min). Peaks were detected by refractive index detector (Waters 2414).

### Results

### Characterization of a Beta-glucosidase from Streptomyces species Substrate specificity

To determine the substrate specificity of the *Streptomyces scabies* beta-glucosidase, the recombinant his-tagged protein was incubated with various cello-oligosaccharides ranging to cellobiose (Glc)₂ to cellohexaose (Glc)₆, and different disaccharides (lactose, saccharose, maltose, threalose and turanose). Samples collected after different incubation times were spotted at the bottom of a thin layer chromatography (TLC) plate and revealed that BglC is able to generate glucose from cellobiose and all cello-oligosaccharides tested (Figure 2). All others disaccharides tested did not reveal hydrolysis by BglC except lactose though with much less efficiency than cellobiose or cello-oligosaccharides (not shown). Kinetic parameters of BglC were determined for the natural substrate cellobiose. Km and kcat values are 0.77 mM and 400 min⁻¹ respectively while the Vmax value is 7.3 µmol.min⁻¹.mg⁻¹.

### Optimal pH and temperature

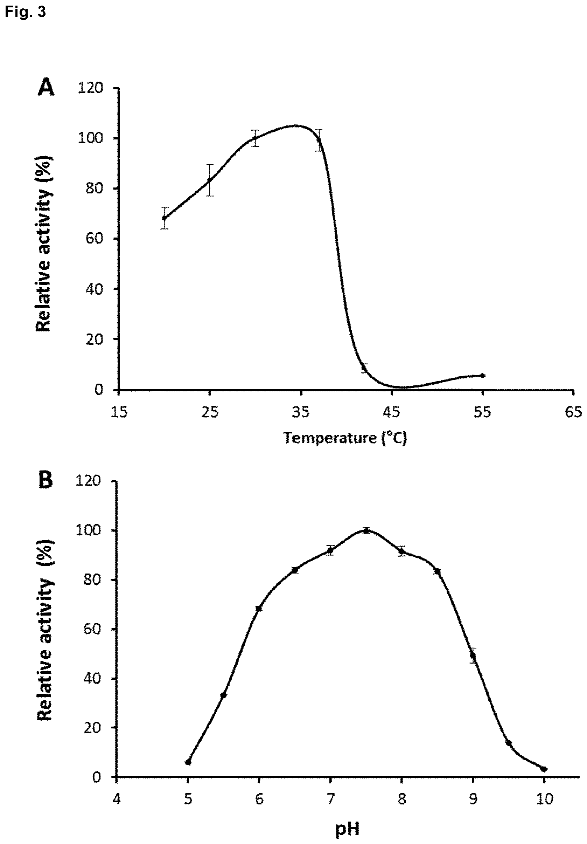

The activity of BglC at different temperatures (20-55°C) and pH (5-10) values was measured using *p*-nitrophenyl-β-D-glucopyranoside (*p*-NPβG) as substrate. The activity of the enzyme gradually increased from 20 to 30°C and display similar activity up to 37°C (Figure 3A). The activity abruptly decline to 10% of its maximal activity at 42°C (Figure 3A). The optimal pH of BglC is around 7.5 and the enzyme conserved high activity when the pH is between 6.5 and 8.5 (Figure 3B). The activity rapidly decline to 30 and 50% of its optimum at pH 5.5 and 9, respectively (Figure 3B).

### Effects of sugars on BglC activity

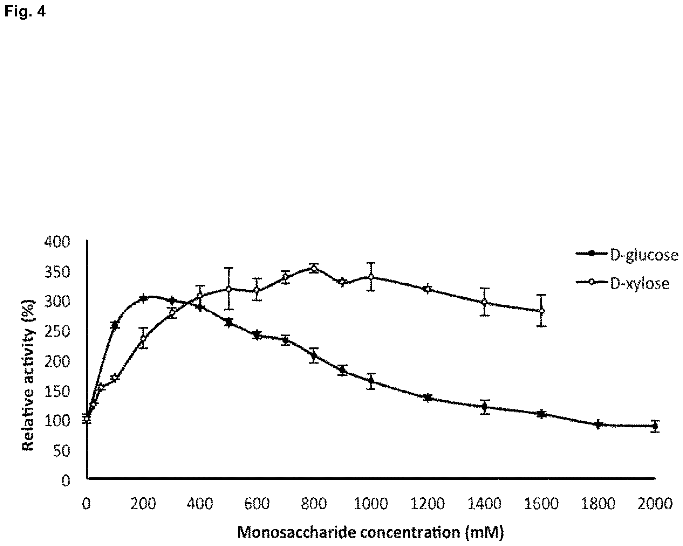

The activity of recombinant BglC was measured in the presence of glucose to assess whether accumulation of the product could inhibit or activate its hydrolytic activity using *p*-NPβG as substrate. The effect of xylose on BglC activity was also assessed, as this sugar, emanating from hemicellulose hydrolysis, is likely to accumulate in processes involved in lignocellulose hydrolytic degradation. Both glucose and xylose revealed to highly enhance the activity of BglC (Figure 4). The activity of BglC increased to around 250-300% when the total concentration of glucose in the assay was ranging from 100 to 700 mM and remained 100% active up to 1.6 M glucose. The addition of xylose better improved the activity of BglC than glucose, with maximal activity of 300-350% measured when the total concentration of xylose was ranging between 0.3 to 1.6 M. Hence BglC from *Streptomyces scabies* is not only glucose- and xylose-tolerant but highly stimulated by these sugars.

In addition, the effect of other sugars on the activity of BglC was further tested (Table 2). Mannose has a moderate effect, and fructose and sorbitol has a similar effect as glucose. The effect of galactose was intermediate between glucose and xylose, the latter being unambiguously the best sugar for enhancing the activity of BgIC.

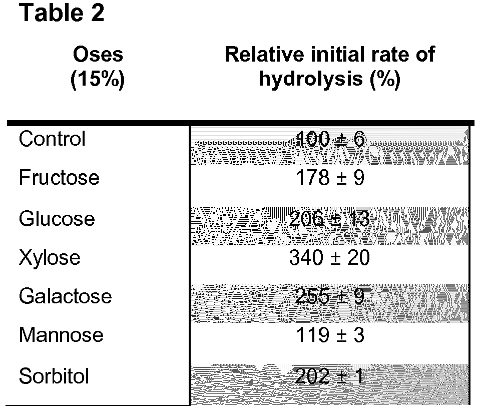

The tolerance of BglC to glucose was further investigated during prolonged reaction time (7 days). Cellobiose and glucose quantification were performed by HPLC (Figure 5). 80% of cellobiose was hydrolyzed in 48h (Figure 5). Glucose was detected as only product generated during the course of the reaction suggesting the absence of transglycosylation between glucose and cellobiose under these conditions. Hence BglC has no or reduced transglycosylation activity.

### Effect of reagents on BglC activity

The effect of several reagents on the activity of BglC under optimal conditions (pH and T°) are shown in Table 3.

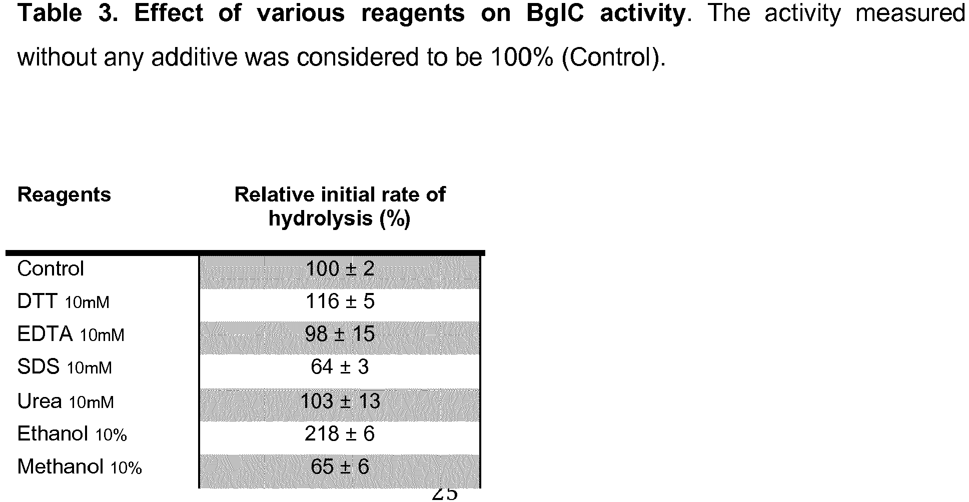

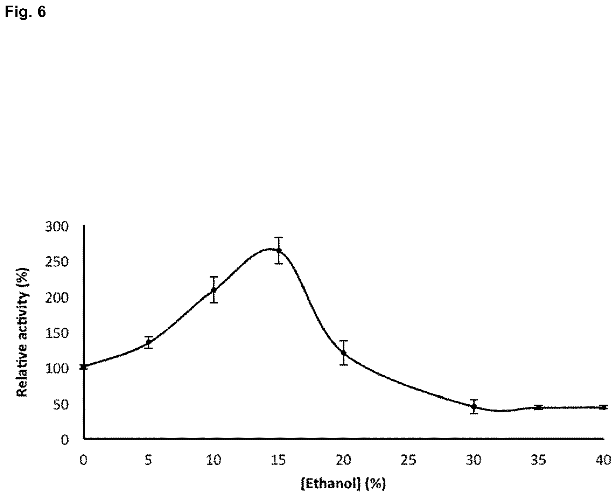

Importantly, ethanol, the final product of the complete enzymatic hydrolysis of cellulose and the subsequent fermentation of glucose, revealed to further enhance the activity of the enzyme. This result was confirmed in assays with various concentrations of ethanol ranging from 0 to 40%, with a maximal enhancing activity detected at 15% (Figure 6).

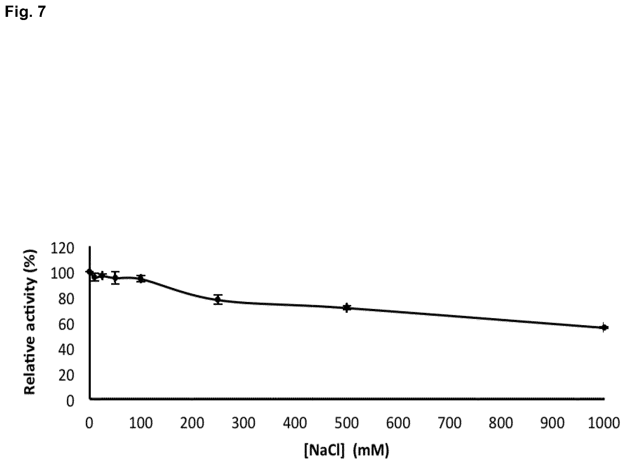

The activity of BglC measured in the presence of 0 to 1000 mM of NaCl reveals that NaCl does not significantly alter the capacity of the enzyme to hydrolyze *p*-NPβG below 100 mM, and that around 70% of the activity is kept at 500 mM (Figure 7). A concentration of 1 M of NaCl was necessary to reduce the activity of BglC to about 50% (Figure 7).

## Claims

1. Use of a polypeptide having sugar-tolerant beta-glucosidase activity for hydrolysis of a lignocellulosic substrate to yield glucose and/or other sugars,
**characterized in that** said polypeptide is selected from the group consisting of:
a) a polypeptide comprising an amino acid sequence at least 50% identical to the amino acid sequence of SEQ ID NO: 2,
b) a polypeptide which is encoded by a polynucleotide at least 50% identical to SEQ ID NO: 1,
c) a polypeptide which is encoded by a polynucleotide which hybridizes under at least medium stringency conditions with the polynucleotide of SEQ ID NO: 1, or a complementary strand thereof, and
d) a fragment of a), b), or c) having beta-glucosidase activity;
and said polypetide or fragment thereof is obtained from a *Streptomyces bacterium.*

2. The use according to claim 1, wherein said polypeptide is activated by sugar.

3. The use according to claims 1 or 2, wherein *Streptomyces* bacterium is preferably *Streptomyces scabies,* more preferably *Streptomyces scabies* 87-22.

4. The use according to any one of claims 1 to 3 , wherein said polypeptide has no or reduced transglycosylation activity.

5. The use according to any one of claims 1 to 4, wherein said polypeptide is further tolerant to one or more of DTT, EDTA, urea or NaCl.

6. The use according to any one of claims 1 to 5, wherein said polypeptide is further tolerant to alcohol, such as ethanol.

7. The use according to any one of claims 1 to 6, wherein said glucose and/or other sugars are further fermented in alcohol, such as ethanol, after hydrolysis.

8. The use according to claim 7, wherein hydrolysis of lignocellulosic substrate and fermentation in alcohol are conducted simultaneously.

9. The use according to any one of claims 1 to 8, wherein said sugar is selected from the group comprising: glucose, xylose, fructose, galactose, mannose, and sorbitol.

10. The use according to any one of claims 1 to 9, wherein said lignocellulosic substrate comprises one or more of cellobiose, cellotriose, cellotetraose, cellopentaose or cellohexaose.

11. Use of a host cell comprising and/or secreting a polypeptide as defined in any one of claims 1 to 5 for hydrolyzing a lignocellulosic substrate in a sugar-tolerant manner, yielding glucose and/or other sugars.

12. The use according to claim 11, wherein said polypeptide further comprises a signal peptide sequence.

13. The use according to claims 11 or 12, wherein said host cell is a bacterial cell or a fungal cell.

14. A kit-of-parts for hydrolyzing a lignocellulosic substrate in a sugar-tolerant manner, comprising: a polypeptide as defined in any one of claims 1 to 5, and one or more other cellulases.

15. The kit-of-parts according to claim 14, wherein the one or more other cellulases are selected from no or one or more other beta-glucosidases, one or more cellobiohydrolases, and one or more endoglucanases.

16. The kit-of-parts according to claim 14 or 15, wherein said sugar is selected from the group comprising: glucose, xylose, fructose, galactose, mannose, and sorbitol.

17. The kit-of-parts according to any one of claims 14 to 16, wherein said lignocellulosic substrate comprises one or more of cellobiose, cellotriose, cellotetraose, cellopentaose or cellohexaose.

## Patentansprüche

1. Verwendung eines Polypeptids mit zuckertoleranter Beta-Glucosidase-Aktivität für die Hydrolyse eines Lignocellulose-Substrats zum Erhalt von Glucose und/oder anderen Zuckern,
**dadurch gekennzeichnet, dass** das Polypeptid ausgewählt ist aus der Gruppe bestehend aus:
a) einem Polypeptid, das eine Aminosäuresequenz umfasst, die wenigstens 50% zur Aminosäuresequenz von SEQ ID NO: 2 identisch ist,
b) einem Polypeptid, das von einem Polynukleotid codiert wird, das wenigstens 50% zu SEQ ID NO: 1 identisch ist,
c) einem Polypeptid, das von einem Polynukleotid codiert wird, das unter wenigstens mittelstringenten Bedingungen mit dem Polynukleotid der SEQ ID NO: 1 oder einem komplementären Strang davon hybridisiert, und
d) einem Fragment von a), b) oder c) mit Beta-Glucosidase-Aktivität;
und wobei das Polypeptid oder Fragment davon aus einem *Streptomyces*-Bakterium gewonnen wird.

2. Verwendung gemäß Anspruch 1, wobei das Polypeptid durch Zucker aktiviert wird.

3. Verwendung gemäß Anspruch 1 oder 2, wobei es sich bei dem *Streptomyces*-Bakterium vorzugsweise um *Streptomyces scabies,* stärker bevorzugt *Streptomyces scabies* 87-22, handelt.

4. Verwendung gemäß einem beliebigen der Ansprüche 1 bis 3, wobei das Polypeptid keine oder eine verringerte Transglycosylierungsaktivität aufweist.

5. Verwendung gemäß einem beliebigen der Ansprüche 1 bis 4, wobei das Polypeptid ferner gegenüber einem oder mehreren von DTT, EDTA, Harnstoff oder NaCl tolerant ist.

6. Verwendung gemäß einem beliebigen der Ansprüche 1 bis 5, wobei das Polypeptid ferner gegenüber Alkohol, wie Ethanol, tolerant ist.

7. Verwendung gemäß einem beliebigen der Ansprüche 1 bis 6, wobei die Glucose und/oder die anderen Zucker nach der Hydrolyse in Alkohol, wie Ethanol, weiter fermentiert werden.

8. Verwendung gemäß Anspruch 7, wobei die Hydrolyse von Lignocellulose-Substrat und die Fermentation in Alkohol gleichzeitig durchgeführt werden.

9. Verwendung gemäß einem beliebigen der Ansprüche 1 bis 8, wobei der Zucker ausgewählt ist aus der Gruppe umfassend: Glucose, Xylose, Fructose, Galactose, Mannose und Sorbitol.

10. Verwendung gemäß einem beliebigen der Ansprüche 1 bis 9, wobei das Lignocellulose-Substrat eines oder mehrere von Cellobiose, Cellotriose, Cellotetraose, Cellopentaose oder Cellohexaose umfasst.

11. Verwendung einer Wirtszelle, die ein Polypeptid, wie in einem beliebigen der Ansprüche 1 bis 5 definiert, umfasst und/oder sezerniert, zum Hydrolysieren eines Lignocellulose-Substrats auf zuckertolerante Weise, wobei Glucose und/oder andere Zucker erhalten werden.

12. Verwendung gemäß Anspruch 11, wobei das Polypeptid ferner eine Signalpeptidsequenz umfasst.

13. Verwendung gemäß Anspruch 11 oder 12, wobei die Wirtszelle eine Bakterienzelle oder eine Pilzzelle ist.

14. Komponenten-Kit zum Hydrolysieren eines Lignocellulose-Substrats auf zuckertolerante Weise, umfassend: ein Polypeptid, wie in einem beliebigen der Ansprüche 1 bis 5 definiert, und eine oder mehrere andere Cellulasen.

15. Komponenten-Kit gemäß Anspruch 14, wobei die eine oder die mehreren anderen Cellulasen ausgewählt sind aus keiner oder einer oder mehreren anderen Beta-Glucosidasen, einer oder mehreren Cellobiohydrolasen und einer oder mehreren Endoglucanasen.

16. Komponenten-Kit gemäß Anspruch 14 oder 15, wobei der Zucker ausgewählt ist aus der Gruppe umfassend: Glucose, Xylose, Fructose, Galactose, Mannose und Sorbitol.

17. Komponenten-Kit gemäß einem beliebigen der Ansprüche 14 bis 16, wobei das Lignocellulose-Substrat eines oder mehrere von Cellobiose, Cellotriose, Cellotetraose, Cellopentaose oder Cellohexaose umfasst.

## Revendications

1. Utilisation d'un polypeptide ayant une activité de bêta-glucosidase tolérante aux sucres, pour l'hydrolyse d'un substrat lignocellulosique afin de conduire à du glucose et/ou d'autres sucres,
**caractérisée en ce que** ledit polypeptide est choisi dans le groupe constitué par :
a) un polypeptide comprenant une séquence d'acides aminés ayant au moins 50 % d'identité avec la séquence d'acides aminés de SEQ ID n° 2,
b) un polypeptide qui est codé par un polynucléotide ayant au moins 50 % d'identité avec SEQ ID n° 1,
c) un polypeptide qui est codé par un polynucléotide qui s'hybride dans des conditions de stringence au moins moyennes avec le polynucléotide de SEQ ID n° 1, ou un brin complémentaire de celui-ci, et
d) un fragment de a), b), ou c) ayant une activité de bêta-glucosidase ;
et ledit polypeptide ou fragment de celui-ci est obtenu à partir d'une bactérie de type *Streptomyces.*

2. Utilisation selon la revendication 1, dans laquelle ledit polypeptide est activé par des sucres.

3. Utilisation selon les revendications 1 ou 2, dans laquelle la bactérie de type *Streptomyces* est préférablement *Streptomyces scabies,* plus préférablement *Streptomyces scabies* 87-22.

4. Utilisation selon l'une quelconque des revendications 1 à 3, dans laquelle ledit polypeptide possède une activité réduite de transglycosylation, voire aucune.

5. Utilisation selon l'une quelconque des revendications 1 à 4, dans laquelle ledit polypeptide est en outre tolérant vis-à-vis d'un ou plusieurs parmi le DTT, l'EDTA, l'urée ou le NaCl.

6. Utilisation selon l'une quelconque des revendications 1 à 5, dans laquelle ledit polypeptide est en outre tolérant vis-à-vis d'un alcool, tel que l'éthanol.

7. Utilisation selon l'une quelconque des revendications 1 à 6, dans laquelle ledit glucose et/ou lesdits autres sucres sont en outre fermentés en alcool, tel que l'éthanol, après hydrolyse.

8. Utilisation selon la revendication 7, dans laquelle l'hydrolyse du substrat lignocellulosique et la fermentation en alcool sont effectuées de manière simultanée.

9. Utilisation selon l'une quelconque des revendications 1 à 8, dans laquelle ledit sucre est choisi dans le groupe constitué par le glucose, le xylose, le fructose, le galactose, le mannose et le sorbitol.

10. Utilisation selon l'une quelconque des revendications 1 à 9, dans laquelle ledit substrat lignocellulosique comprend un ou plusieurs parmi le cellobiose, le cellotriose, le cellotétraose, le cellopentaose ou le cellohexaose.

11. Utilisation d'une cellule hôte comprenant et/ou sécrétant un polypeptide tel que défini selon l'une quelconque des revendications 1 à 5, pour l'hydrolyse d'un substrat lignocellulosique de manière tolérante aux sucres, conduisant à du glucose et/ou d'autres sucres.

12. Utilisation selon la revendication 11, dans laquelle ledit polypeptide comprend en outre une séquence de peptide signal.

13. Utilisation selon les revendications 11 ou 12, dans laquelle ladite cellule hôte est une cellule bactérienne ou une cellule fongique.

14. Kit de parties pour l'hydrolyse d'un substrat lignocellulosique de manière tolérante aux sucres, comprenant un polypeptide tel que défini selon l'une quelconque des revendications 1 à 5, et une ou plusieurs autres cellulases.

15. Kit de parties selon la revendication 14, dans lequel les une ou plusieurs autres cellulases sont choisies parmi aucune ou une ou plusieurs bêta-glucosidases, une ou plusieurs cellobiohydrolases, et une ou plusieurs endoglucanases.

16. Kit de parties selon la revendication 14 ou 15, dans lequel ledit sucre est choisi dans le groupe constitué par le glucose, le xylose, le fructose, le galactose, le mannose et le sorbitol.

17. Kit de parties selon l'une quelconque des revendications 14 à 16, dans lequel ledit substrat lignocellulosique comprend un ou plusieurs parmi le cellobiose, le cellotriose, le cellotétraose, le cellopentaose ou le cellohexaose.


### SEQUENCE LISTING

<110> Universite de Liege
<120> USE OF A SUGAR TOLERANT BETA-GLUCOSIDASE
<130> 2015-34
<160> 2
<170> BiSSAP 1.2
<210> 1
   <211> 1443
   <212> DNA
   <213> Streptomyces scabiei
<220>
   <221> source
   <222> 1..1443
   <223> /organism="Streptomyces scabiei" /mol_type="unassigned DNA"
<400> 1
   atgcctgaac ccgtgaatcc ggccaccccg gtgacctttc ctcccgcctt cctctggggc      60
   gcggccacct ccgcgtacca gatcgagggg gcggtgcggg aggacggccg tacgccctcc     120
   atctgggaca ccttcagtca cacgccgggc aagaccgccg gcggcgagaa cggtgacatc     180
   gctgtcgacc actaccaccg ctaccgcgac gacgtggcga tgatggcgga cctgggcctc     240
   aacgcgtacc gcttctccgt ctcctggtcg cgggtgcagc cgacggggcg gggcccggcc     300
   gtccagaagg ggctcgactt ctaccgacgg ctggtcgacg agctgctggc caagggcatc     360
   aagcccgccg tcaccctcta ccactgggac ctcccgcagg agctggagga cgccggcggc     420
   tggcccgagc gggacatcgt gcaccggttc gccgagtacg cgcggatcat gggcgaggcg     480
   ctcggcgacc gcgtcgagca gtggatcacc ctcaacgagc cgtggtgcac cgcgttcctg     540
   ggctacggct ccggggtgca cgcgccgggc cgtacggacc cggtggcgtc cctgcgcgcg     600
   gcccaccatc tgaacgtggc gcacggcctc ggcgtctcgg cgctgcggtc ggcgatgccc     660
   gcccgcaact cgatcgcggt gagcctcaac tcctcggtgg tgcggccgat caccagctcc     720
   ccggaggacc gggccgcggc ccggaagatc gacgacctcg cgaacggcgt cttccacgga     780
   ccgatgctgc acggggccta cccggagacc ctgttcgccg cgacctcgtc gctgacggac     840
   tggtcgttcg tgcgggacgg tgacgtggcg acggcccatc agccgctgga cgctctgggg     900
   ctgaactact acacgccggc gctggtcggc gcggcggacg ccggcctgga gggcccccgc     960
   gcggacggcc acggggcgag cgagcactcg ccgtggccgg ccgcggacga cgtcctgttc    1020
   caccagaccc cgggcgagcg tacggagatg ggctggacca tcgacccgac gggcctgcac    1080
   gagctgatca tgcggtacgc gcgggaggct ccgggcctgc cgatgtacgt gacggagaac    1140
   ggcgccgcgt acgacgacaa gatggacgcg gacggccgtg tccacgaccc cgagcgcatc    1200
   gcctacctgc acggccacct gcgggcggtc cggcgcgcga tcgccgaggg ggcggacgtg    1260
   cgcgggtact acctgtggtc cctgatggac aacttcgagt gggcgtacgg ctacggcaag    1320
   cgcttcggcg cggtgtacgt cgactacgcg accctgaccc gcacaccgaa gtcgagcgcg    1380
   cactggtacg ggcaggcggc gaagacgggc gccctcccgc cgctggcgcc ggcgccggcg    1440
   tag                                                                  1443
<210> 2
   <211> 480
   <212> PRT
   <213> Streptomyces scabiei
<400> 2
   Met Pro Glu Pro Val Asn Pro Ala Thr Pro Val Thr Phe Pro Pro Ala
   1               5                   10                  15
   Phe Leu Trp Gly Ala Ala Thr Ser Ala Tyr Gln Ile Glu Gly Ala Val
               20                  25                  30
   Arg Glu Asp Gly Arg Thr Pro Ser Ile Trp Asp Thr Phe Ser His Thr
           35                  40                  45
   Pro Gly Lys Thr Ala Gly Gly Glu Asn Gly Asp Ile Ala Val Asp His
       50                  55                  60
   Tyr His Arg Tyr Arg Asp Asp Val Ala Met Met Ala Asp Leu Gly Leu
   65                  70                  75                  80
   Asn Ala Tyr Arg Phe Ser Val Ser Trp Ser Arg Val Gln Pro Thr Gly
                   85                  90                  95
   Arg Gly Pro Ala Val Gln Lys Gly Leu Asp Phe Tyr Arg Arg Leu Val
               100                 105                 110
   Asp Glu Leu Leu Ala Lys Gly Ile Lys Pro Ala Val Thr Leu Tyr His
           115                 120                 125
   Trp Asp Leu Pro Gln Glu Leu Glu Asp Ala Gly Gly Trp Pro Glu Arg
       130                 135                 140
   Asp Ile Val His Arg Phe Ala Glu Tyr Ala Arg Ile Met Gly Glu Ala
   145                 150                 155                 160
   Leu Gly Asp Arg Val Glu Gln Trp Ile Thr Leu Asn Glu Pro Trp Cys
                   165                 170                 175
   Thr Ala Phe Leu Gly Tyr Gly Ser Gly Val His Ala Pro Gly Arg Thr
               180                 185                 190
   Asp Pro Val Ala Ser Leu Arg Ala Ala His His Leu Asn Val Ala His
           195                 200                 205
   Gly Leu Gly Val Ser Ala Leu Arg Ser Ala Met Pro Ala Arg Asn Ser
       210                 215                 220
   Ile Ala Val Ser Leu Asn Ser Ser Val Val Arg Pro Ile Thr Ser Ser
   225                 230                 235                 240
   Pro Glu Asp Arg Ala Ala Ala Arg Lys Ile Asp Asp Leu Ala Asn Gly
                   245                 250                 255
   Val Phe His Gly Pro Met Leu His Gly Ala Tyr Pro Glu Thr Leu Phe
               260                 265                 270
   Ala Ala Thr Ser Ser Leu Thr Asp Trp Ser Phe Val Arg Asp Gly Asp
           275                 280                 285
   Val Ala Thr Ala His Gln Pro Leu Asp Ala Leu Gly Leu Asn Tyr Tyr
       290                 295                 300
   Thr Pro Ala Leu Val Gly Ala Ala Asp Ala Gly Leu Glu Gly Pro Arg
   305                 310                 315                 320
   Ala Asp Gly His Gly Ala Ser Glu His Ser Pro Trp Pro Ala Ala Asp
                   325                 330                 335
   Asp Val Leu Phe His Gln Thr Pro Gly Glu Arg Thr Glu Met Gly Trp
               340                 345                 350
   Thr Ile Asp Pro Thr Gly Leu His Glu Leu Ile Met Arg Tyr Ala Arg
           355                 360                 365
   Glu Ala Pro Gly Leu Pro Met Tyr Val Thr Glu Asn Gly Ala Ala Tyr
       370                 375                 380
   Asp Asp Lys Met Asp Ala Asp Gly Arg Val His Asp Pro Glu Arg Ile
   385                 390                 395                 400
   Ala Tyr Leu His Gly His Leu Arg Ala Val Arg Arg Ala Ile Ala Glu
                   405                 410                 415
   Gly Ala Asp Val Arg Gly Tyr Tyr Leu Trp Ser Leu Met Asp Asn Phe
               420                 425                 430
   Glu Trp Ala Tyr Gly Tyr Gly Lys Arg Phe Gly Ala Val Tyr Val Asp
           435                 440                 445
   Tyr Ala Thr Leu Thr Arg Thr Pro Lys Ser Ser Ala His Trp Tyr Gly
       450                 455                 460
   Gln Ala Ala Lys Thr Gly Ala Leu Pro Pro Leu Ala Pro Ala Pro Ala
   465                 470                 475                 480